# EUROPEAN PATENT SPECIFICATION

(11) **EP 0 783 523 B1**
(45) Date of publication and mention of the grant of the patent: **20.10.2004**
(21) Application number: 94932175.6
(22) Date of filing: 26.08.1994
(51) Int. Cl.: C12N 15/12, C07K 14/705, C07K 16/28, A61K 38/17, C12Q 1/68

(54) **GABA A RECEPTOR EPSILON SUBUNIT**
EPSILON-UNTEREINHEIT DES GABA A-REZEPTORS
SOUS-UNITE EPSILON DE RECEPTEUR GABA A

(43) Date of publication of application: 16.07.1997
(73) Proprietor: HUMAN GENOME SCIENCES, INC., Rockville, MD 20850-3338 (US)
(72) Inventor: LI, Yi, Gaithersburg, MD 20878 (US); KIRKNESS, Ewen, Olney, MD 20832 (US)
(74) Representative: Jaenichen, Hans-Rainer, Dr.
(86) International application number: PCT/US1994/009589
(87) International publication number: WO 1996/006862

(56) References cited:
- US-A- 5 166 066
- Z. ZHAO ET AL: "Isolation of distantly related memebers in a multigene family using the polymerase chain reaction technique" BIOCHEMICAL AND BIOPHYSICAL RESEARCH COMMUNICATIONS., vol. 167, no. 1, 28 February 1990, pages 174-182, XP002075065 ORLANDO, FL US
- PROC. NATL. ACAD. SCI. U.S.A., Vol. 87, issued December 1990, P. WHITING et al., "Another Mechanism For Creating Diversity in Gamma-Aminobutyrate Type A Receptors: RNA Splicing Directs Expression of Two Forms of Gamma2 Subunit, One of Which Contains a Protein Kinase C Phosphorylation Site", pages 9966-9970.
- THE JOURNAL OF BIOLOGICAL CHEMISTRY, Vol. 267, No. 12, issued 25 April 1992, M.H. BUREAU et al., "Isolation and Cloning of a Voltage-Dependant Anion Channel-Like Mr 36,000 Polypeptide From Mammalian Brain", pages 8679-8684.
- THE EMBO JOURNAL, Vol. 9, No. 10, issued 1990, S. YMER et al., "Structural and Functional Characterization of the Gamma1 Subunit of GABAA/Benzodiazepine Receptors", pages 3261-3267.

## Description

This invention relates to newly identified polynucleotides, polypeptides encoded by such polynucleotides, the use of such polynucleotides and polypeptides, as well as the production of such polynucleotides and polypeptides. More particularly, the polypeptide of the present invention is a human GABA_{A} receptor. The invention also relates to inhibiting the action of such polypeptides.

Gamma-aminobutyric acid (GABA) is the major inhibitory neurotransmitter in the mammalian central nervous system. The major type of receptor for the inhibitory neurotransmitter GABA, called the GABA_{A} receptor (GABA_{A}), is a member of a gene superfamily of ligand-gated ion channels. GABA, the endogenous ligand for the GABA_{A} complex, stimulates chloride ion conductance through the associated chloride ion channel. The predominant effect of GABA is the interaction with a specific receptor protein which results in an increase of the chloride ion conductance of the post-synaptic membrane to produce an inhibition of neuronal firing. GABA is found in many central neurons (e.g., basal ganglia, cerebellum). GABA is derived from glutamate, which is decarboxylated by glutamic acid decarboxylase. After interaction with receptors, GABA is actively "pumped" back into the prejunctional neurons.

The GABA_{A} receptor is a multi-subunit ligand-gated ion channel. This receptor is a heterooligomeric protein composed of several distinct polypeptide types. Five different classes of subunit have been defined, α, β, γ₁, δ_{λ} and ρ. Molecular cloning of these polypeptides reveals that they show 20-40% identity with each other, and 10-20% identity with polypeptides of the nicotinic acetylcholine receptors and strychnine-sensitive glycine receptor. Each subunit class is also represented by a family of genes whose members have 60-80% amino acid sequence identity. Sequences of 6 α, 3 β, 3 γ, 1 δ and 2 ρ subunits have been reported. Regions of conserved and variable amino acid sequence suggests structural and functional domains within each polypeptide. All of the polypeptides when expressed in heterologous cells produce GABA-activated chloride channels, and different subunit combinations express different pharmacological properties. The distributions of mRNAs for the different GABA_{A} receptor polypeptides and their subtypes show significant brain regional variation consistent with pharmacological and biochemical evidence for receptor heterogeneity. Subpopulations of GABA_{A} receptors with different cellular and regional locations show different sensitivity to GABA, to modulators like steroids, to physiological regulation, to disease processes and to pharmacological manipulation by drugs such as benzodiazepines. The properties of the different subpopulations of GABA_{A} receptors are determined by which of the one or more different subunits are expressed in a given cell to produce a variety of different oligomeric protein structures.

The GABA_{A} receptor chloride-ionophore complex is the primary site of action for many of the drugs used to treat anxiety and seizure disorders such as the benzodiazepines and anticonvulsant barbiturates. By allosteric drug-induced modulation the receptors serve as molecular control elements through which the levels of anxiety, vigilance, muscle tension and epileptic activity can be regulated.

Whiting et al. (Proc. Natl. Acad. Sci. USA 87 (1990), 9966-9970) describes that diversity in γ-aminobutyrate type A receptors can be the result of alternative RNA splicing. Bureau et al. (J. Biol. Chem. 267 (1992), 8679-8684) describes the isolation and cloning of a 36 kDa polypeptide from mammalian brain which was identified as being a new member of the voltage dependent anion channel (VDAC) family of proteins. Ymer et al. (EMBO J. 9 (1990), 3261-3267) discloses the structural and functional characterization of the γ₁ subunit of GABA_{A}/benzodiazepine receptors. Zhao and Joho (Biochem. Biophys. Res. Comm. 167 (1990), 174-182) report on the isolation of distantly related members in gene families by applying the polymerase chain reaction technique. The described method is exemplified by using subunits of the GABA_{A} receptor. US Patent 5,166,066 discloses stable transformed HEK 293 cells comprising a functional GABA_{A} receptor that comprises an α, β and γ GABA_{A} receptor subunit.

The present inventors have found a new class of GABA_{A} subunit. This new GABA_{A} subunit may be responsible for similar and additional pharmacological events as the other classes of GABA_{A} subunit.

In accordance with one aspect of the present invention, there is provided a novel mature polypeptide which is a GABA_{A} receptor epsilon subunit, as well as fragments, analogs and derivatives thereof. The polypeptide of the present invention is of human origin.

In accordance with another aspect of the present invention, there are provided polynucleotides (DNA or RNA) which encode such polypeptides.

In accordance with yet a further aspect of the present invention, there is provided a process for producing such polypeptide by recombinant techniques.

In accordance with yet a further aspect of the present invention, there are provided agonists against such GABA_{A} receptor epsilon subunits and a pharmaceutical composition comprising such agonists for therapeutic purposes, for example, to diagnose and treat anxiety, Huntington's Chorea and sleep and seizure disorders.

In accordance with yet a further aspect of the present invention, there are provided antibodies against such polypeptides.

In accordance with yet another aspect of the present invention, there are provided antagonists to such polypeptides, which may be used to inhibit the action of such polypeptides, for example, in the treatment of Alzheimer's disease, Parkinson's disease, overdoses with benzodiazepine drugs, and other neurological disorders and to enhance memory.

These and other aspects of the present invention should be apparent to those skilled in the art from the teachings herein.

The following drawings are illustrative of embodiments of the invention and are not meant to limit the scope of the invention as encompassed by the claims.

Figure 1 shows the cDNA and corresponding deduced amino acid sequence of GABA_{A} receptor epsilon subunit. The full length protein is 440 amino acid residues with the first 24 amino acids representing the putative leader sequence such that the mature protein comprises 416 amino acid residues.

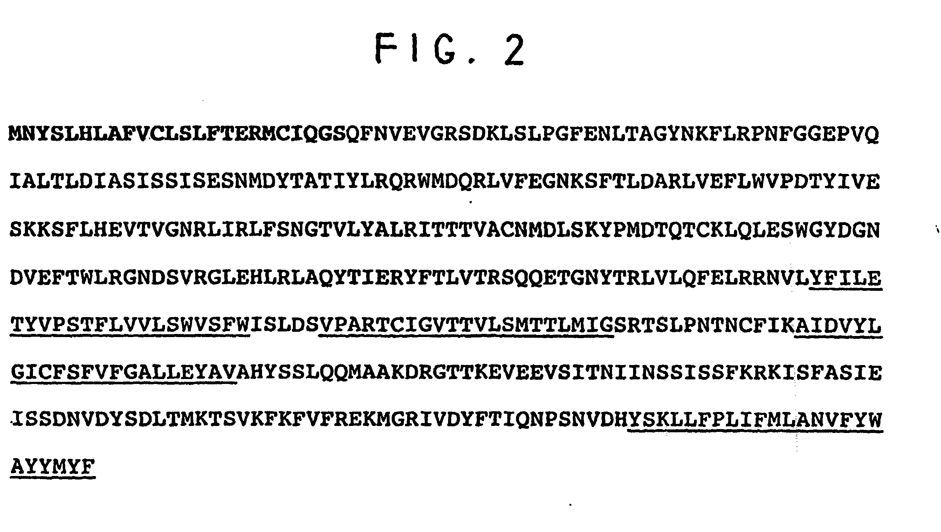

Figure 2 illustrates the amino acid sequence for GABA_{A} receptor epsilon subunit where the standard one letter abbreviation for amino acids is used. The putative transmembrane domains are underlined.

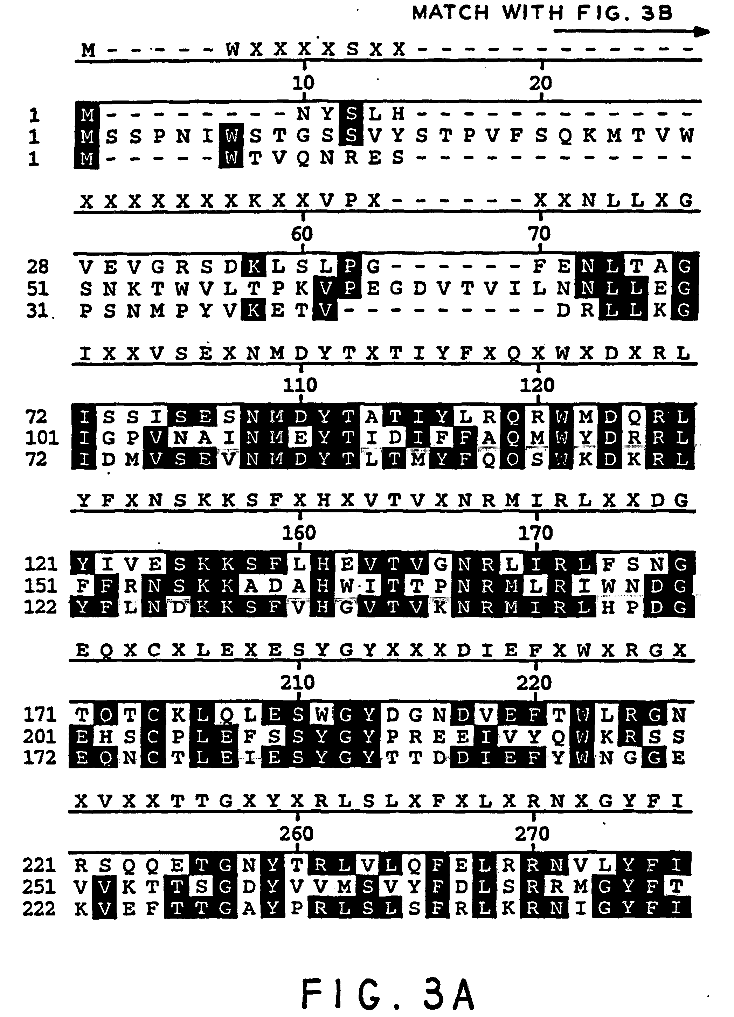

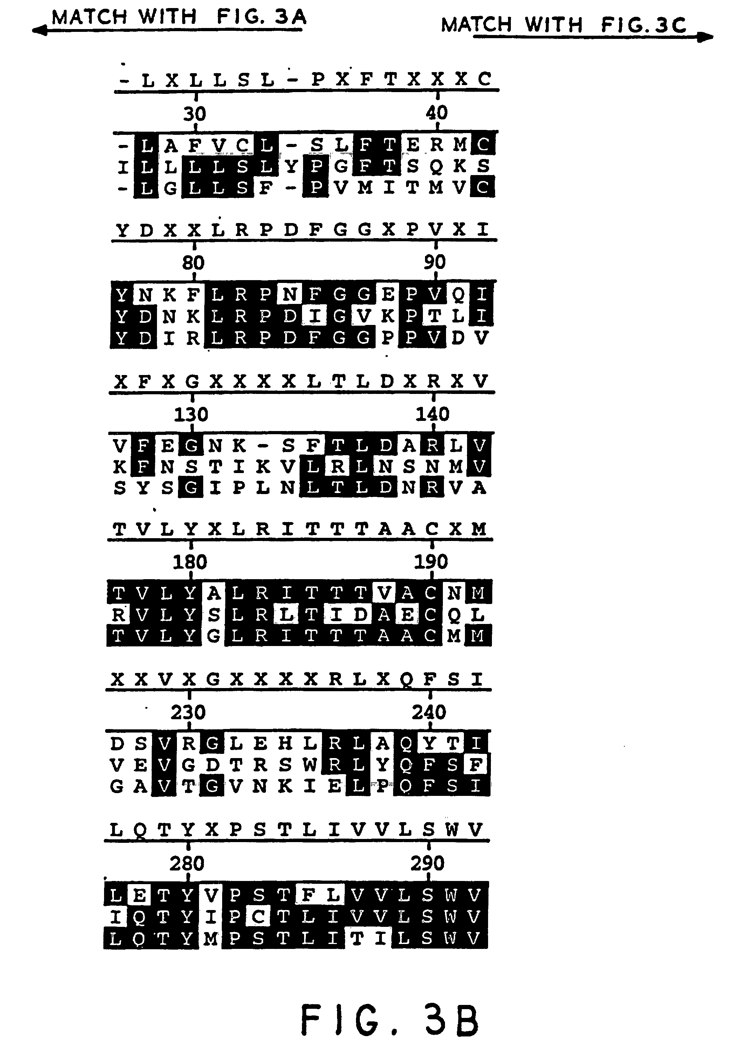

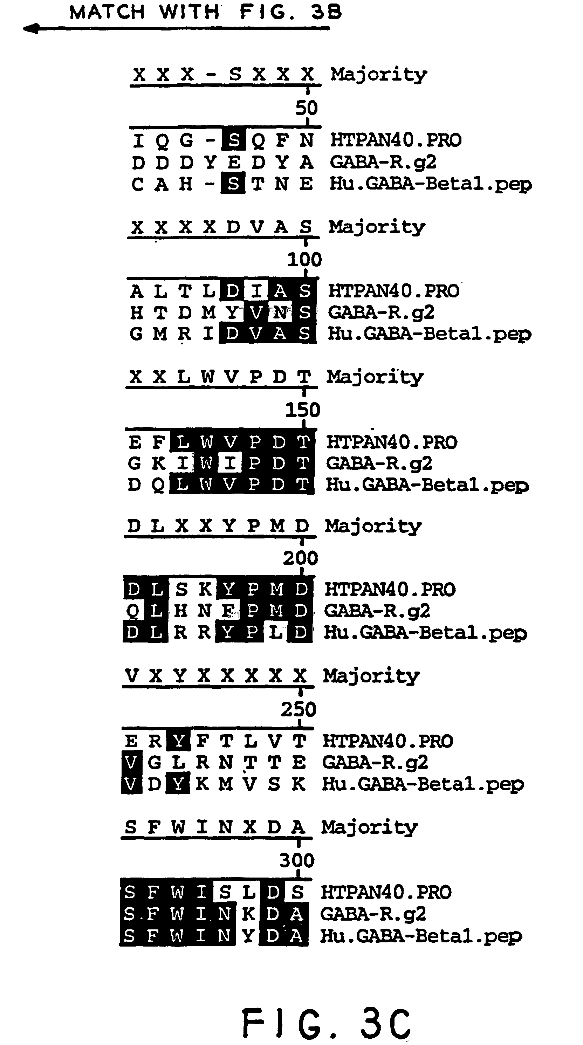

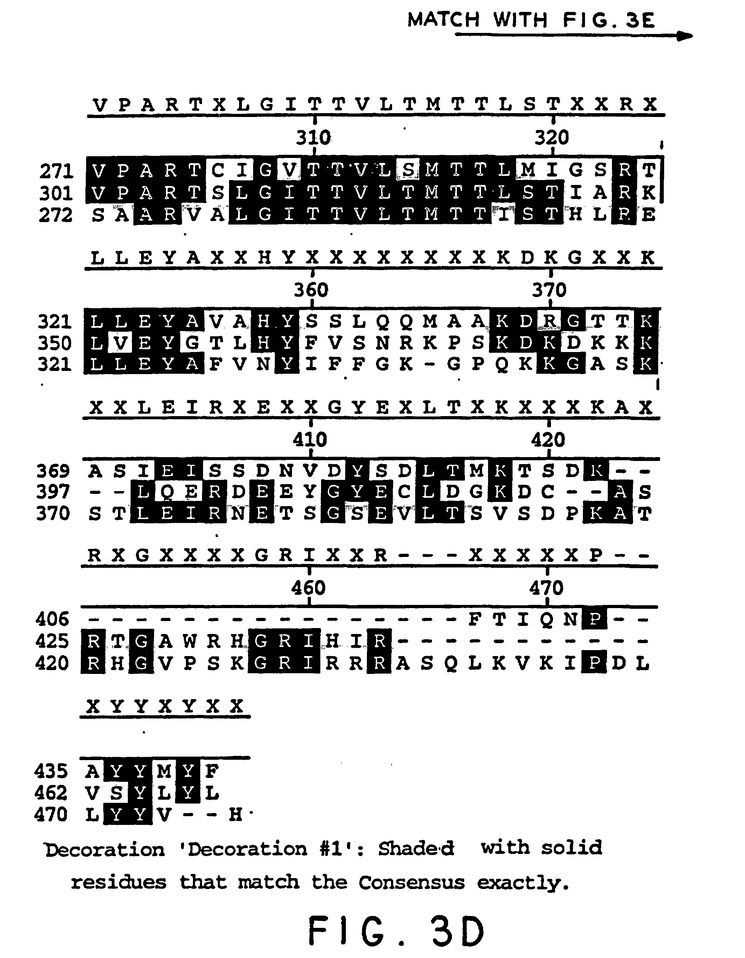

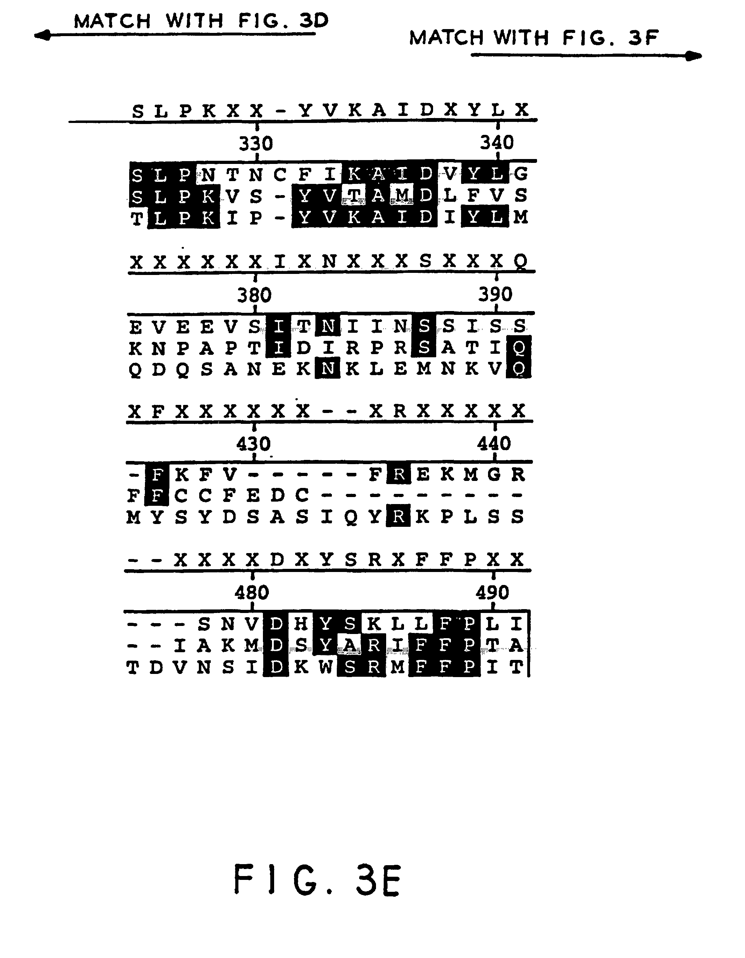

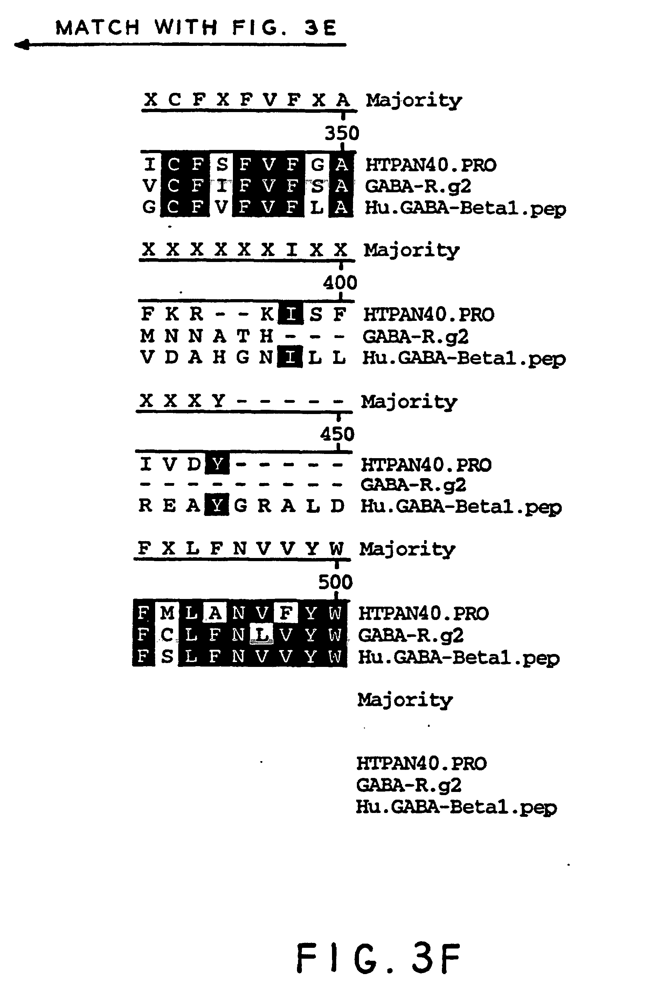

Figure 3 shows an amino acid sequence comparison of the GABA_{A} receptor epsilon subunit with other GABA_{A} receptor subunits. The shaded residues are those that match exactly.

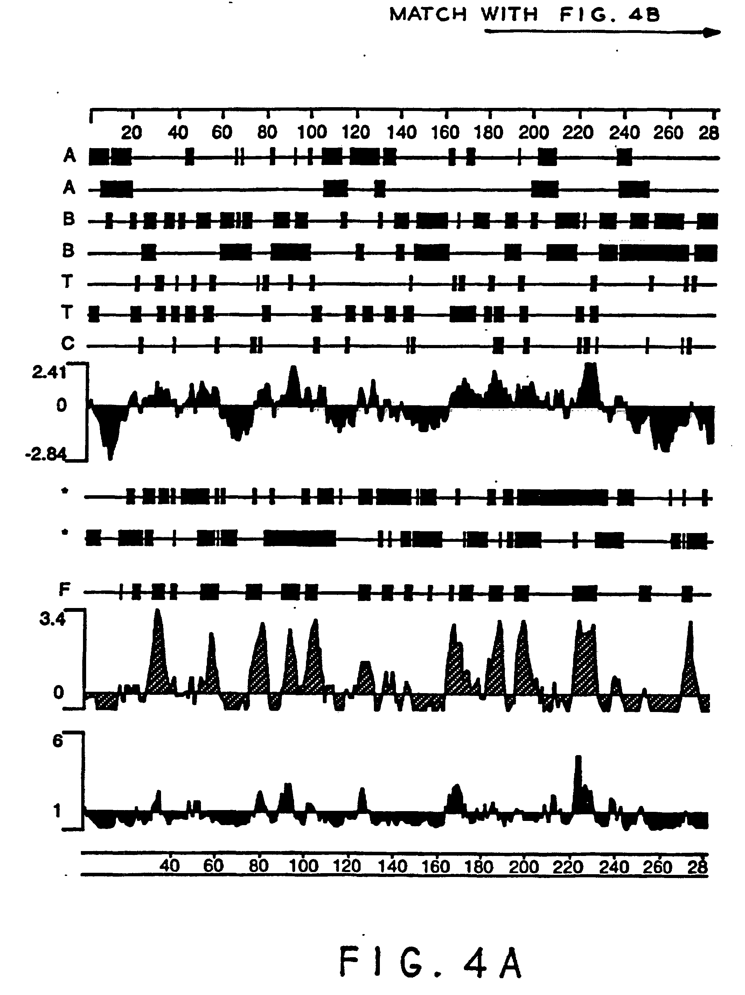

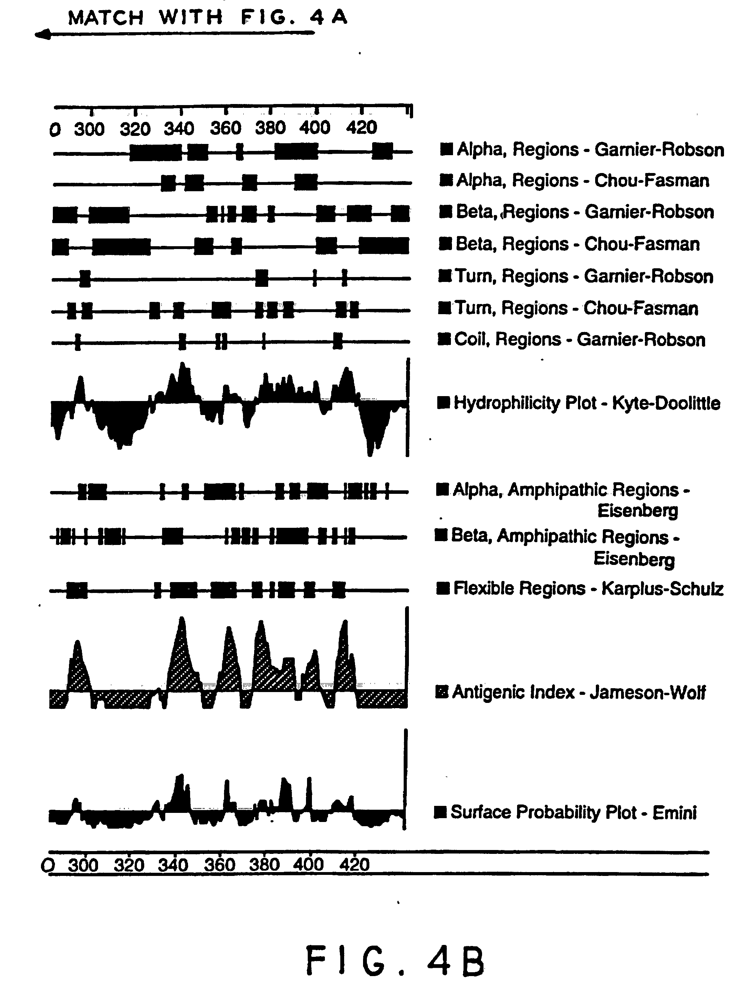

Figure 4 is an illustration of the secondary structural features of the GABA_{A Epsilon Recep}tor Subunit. The first 7 illustrations set forth the regions of the amino acid sequence which are alpha helices, beta sheets, turn regions or coiled regions. The boxed areas are the areas which correspond to the region indicated. The second set of figures illustrate areas of the amino acid sequence which are exposed to intracellular, cytoplasmic or are membrane-spanning. The hydrophilicity plot illustrates areas of the protein sequence which are the lipid bilayer of the membrane and are, therefore, hydrophobic, and areas outside the lipid bilayer membrane which are hydrophilic. The antigenic index corresponds to the hydrophilicity plot, since antigenic areas are areas outside the lipid bilayer membrane and are capable of binding antigens. The surface probability plot further corresponds to the antigenic index and the hydrophilicity plot. The amphipathic plots show those regions of the protein sequences which are polar and non-polar. The flexible regions correspond to the second set of illustrations in the sense that flexible regions are those which are outside the membrane and inflexible regions are transmembrane regions.

The present invention relates to a polynucleotide selected from the group consisting of
(a) polynucleotides encoding at least the mature form of the polypeptide having the deduced amino acid sequence as shown in SEQ ID NO:2;
(b) polynucleotides having the coding sequence as shown in SEQ ID NO:1 encoding at least the mature form of the polypeptide;
(c) polynucleotides encoding the polypeptide having the amino acid sequence of at least the mature form of the polypeptide encoded by the cDNA contained in ATCC 75810;
(d) polynucleotides having the coding sequence of the cDNA contained in ATCC 75810 encoding at least the mature form of the polypeptide;
(e) polynucleotides encoding a fragment of a polypeptide encoded by a polynucleotide of any one of (a) to (d) wherein said fragment has the activity of a polypeptide as encoded by a polynucleotide of any one of (a) to (d) and/or binds an antibody that specifically binds to a polypeptide having the sequence as shown in SEQ ID NO:2; and
(f) polynucleotides the sequence of which has 95% sequence identity to a polynucleotide as defined in any one of (a) to (d);
or the complementary strand of such a polynucleotide.

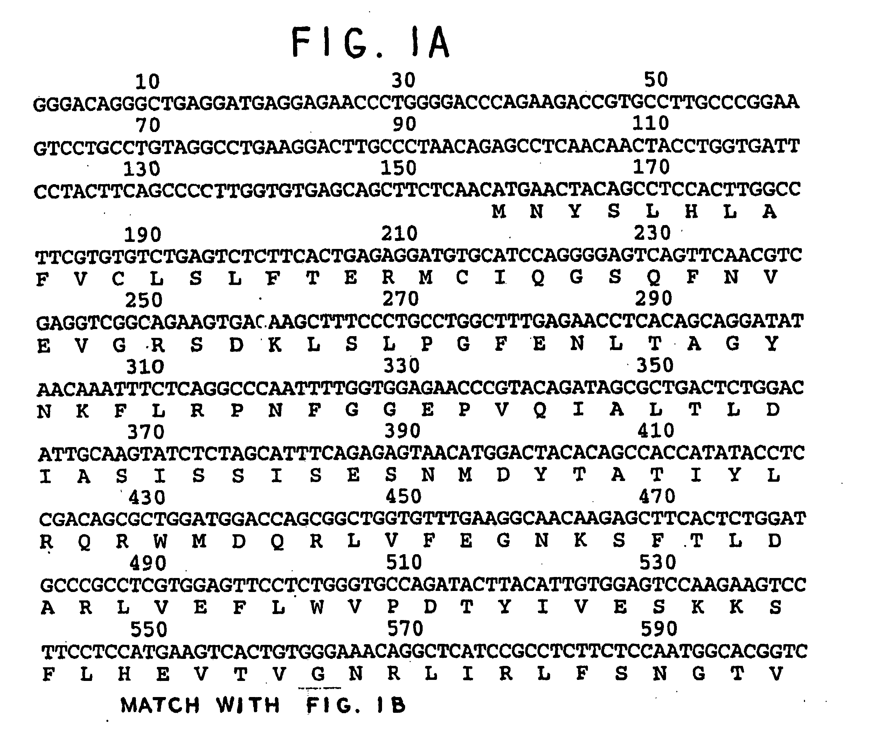

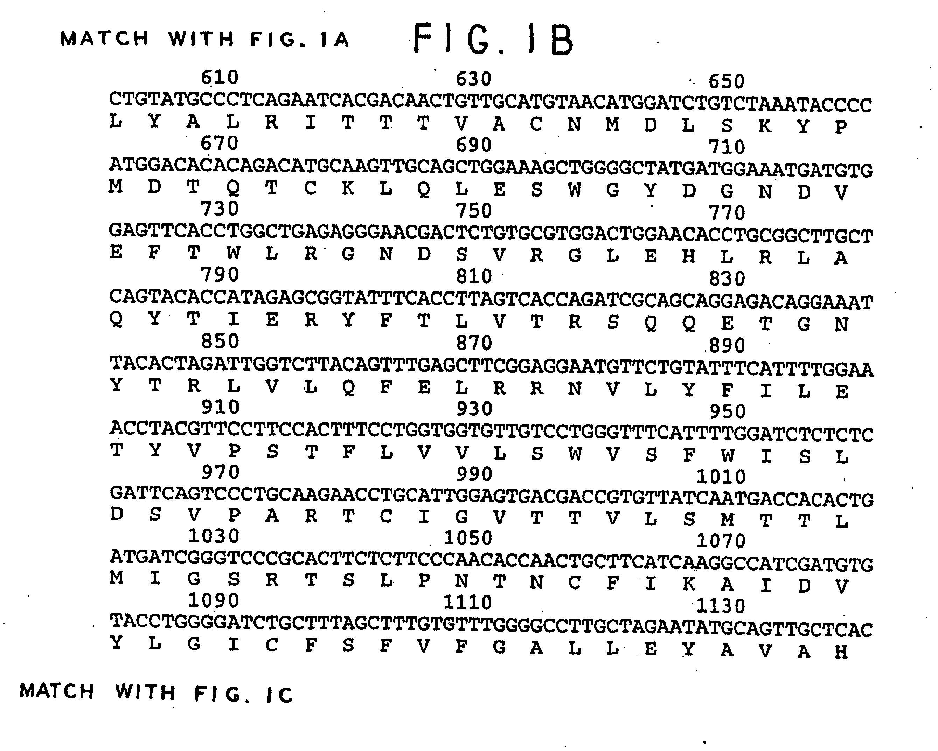

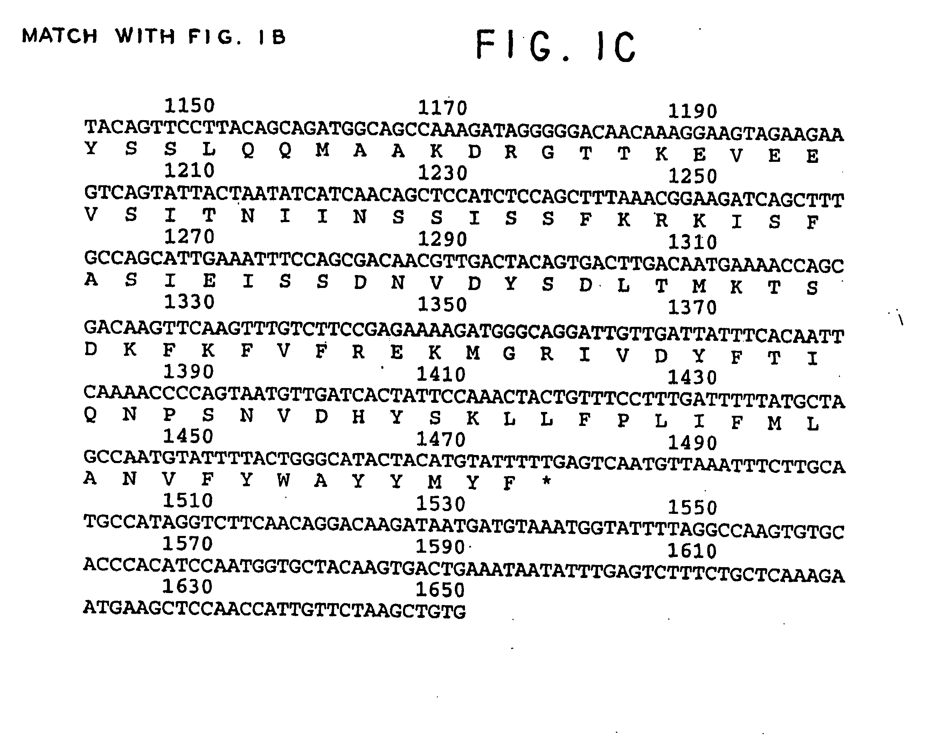

In accordance with an aspect of the present invention, there is provided an isolated nucleic acid (polynucleotide) which encodes for the mature polypeptide having the deduced amino acid sequence of Figure 1 for the mature polypeptide encoded by the cDNA of the clone deposited as ATCC Deposit No. 75810 on June 10,1994.

The polynucleotide of this invention was discovered in a cDNA library derived from pancreas tumor. It is structurally related to the GABA_{A} subunit receptor family. It contains an open reading frame encoding a protein of about 440 amino acid residues of which approximately the first 24 amino acids residues are the putative leader sequence such that the mature protein comprises 416 amino acids. The protein exhibits the highest degree of homology to rat GABA_{A} receptor epsilon Beta-1 subunit with 50 % identity and 70 % similarity over a 400 amino acid stretch.

The polynucleotide of the present invention may be in the form of RNA or in the form of DNA, which DNA includes cDNA, genomic DNA, and synthetic DNA. The DNA may be doublestranded or single-stranded, if single stranded may be the coding strand or non-coding (anti-sense) strand. The coding sequence which encodes the mature polypeptide may be identical to the coding sequence shown in Figure 1 or that of the deposited clone or may be a different coding sequence which coding sequence, as a result of the redundancy or degeneracy of the genetic code, encodes the same mature polypeptide as the DNA of Figure 1 or the deposited cDNA.

The polynucleotide which encodes for the mature polypeptide of Figure 1 or for the mature polypeptide encoded by the deposited cDNA may include: only the coding sequence for the mature polypeptide; the coding sequence for the mature polypeptide and additional coding sequence such as a leader or secretory sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments of the polypeptide having the deduced amino acid sequence of Figure 1 or the polypeptide encoded by the cDNA of the deposited clone.

Thus, the present invention includes polynucleotides encoding the same mature polypeptide as shown in Figure 1 or the same mature polypeptide encoded by the cDNA of the deposited clone as well as variants of such polynucleotides which variants encode for a fragment of the polypeptide of Figure 1 or the polypeptide encoded by the cDNA of the deposited clone.

The polynucleotide may have a coding sequence which is a naturally occurring allelic variant of the coding sequence shown in Figure 1 or of the coding sequence of the deposited clone. As known in the art, an allelic variant is an alternate form of a polynucleotide sequence which may have a substitution, deletion or addition of one or more nucleotides, which does not substantially alter the function of the encoded polypeptide.

The present invention also includes polynucleotides, wherein the coding sequence for the mature polypeptide may be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide.

The polynucleotides of the present invention may also have the coding sequence fused in frame to a marker sequence which allows for purification of the polypeptide of the present invention. The marker sequence may be a hexahistidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 50% and preferably 70% identity between the sequences. The present invention particularly relates to polynucleotides which hybridize under stringent conditions to the hereinabove-described polynucleotides . As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which retain substantially the same biological function or activity as the mature polypeptide encoded by the cDNA of Figure 1 or the deposited cDNA. Such hybridizing polynucleotides include deletion variants, substitution variants and addition or insertion variants of the above-described polynucleotides.

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to a GRBA_{A} epsilon subunit polypeptide which has the deduced amino acid sequence of Figure 1 or which has the amino acid sequence encoded by the deposited cDNA, as well as fragments, analogs and derivatives of such polypeptide.

The term "fragment" when referring to the polypeptide of Figure 1 or that encoded by the deposited cDNA, means a polypeptide which retains essentially the same biological function or activity as such polypeptide. The present invention also relates to polynucleotides which encode a proprotein which can be activated by cleavage of the proprotein portion to produce an active mature polypeptide.

The polypeptide of the present invention may be a recombinant polypeptide, a natural polypeptide or a synthetic polypeptide, preferably a recombinant polypeptide.

The present invention also relates to fragments or variants of the polypeptide of Figure 1 or that encoded by the deposited cDNA which may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments or variants are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the GABA_{A} epsilon subunit genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Stregtomyces, Salmonella typhimurium; fungal cells, such as yeast; insect cells such as Drosophila and Sf9; animal cells such as CHO, COS or Bowes melanoma; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pbs, pD10, phagescript, psiX174, pbluescript SK, pbsks, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a.lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation. (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operous encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The GABA_{A} epsilon subunit polypeptide can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the resent invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

The GABA_{A} epsilon subunit polypeptide of the present invention is also useful for identifying other subunits of the GABA_{A} receptor. An example of a procedure for identifying these subunits comprises raising high titer polyclonal antisera against unique, bacterially expressed GABA_{A} epsilon polypeptides. These polyclonal antisera are then used to immunoprecipitate detergent-solubilized GABA_{A} receptors from a mammalian brain preferably a rat brain. Also, preferably the hippocampus section of the brain is used.

The GABA_{A} epsilon polypeptide may also be used to screen ligand/modulators of receptor function. This screen is particularly useful for identifying agonists and antagonists of the GABA_{A} epsilon subunit receptor. An example of such an assay is the patch clamp assay. The DNA encoding the GABA_{A} epsilon subunit is inserted into a HEK 293 cell. The DNA sequence is inserted into an expression cassette, preferably into an expression plasmid. As used herein "expression cassette" refers to DNA sequences necessary for expression of coding sequence in eukaryotic cells. An example of a plasmid is pCDM8 which is commercially available and preferred.

Plasmids containing a selectable marker gene may be co-transfected with plasmids containing DNA sequences that encode the GABA_{A} epsilon subunit. GABA_{A} receptors are gated channels which, upon binding GABA, allow chloride ions to pass into the cell. The GABA_{A} receptor will allow GABA to open the channel and let more chloride into the cell if the molecule being tested is an agonist or less chloride ions into the cell if the molecule being tested is an antagonist. The amount of chloride ions that pass through the GABA_{A} receptor epsilon subunit can be directly measured using the patch clamp assay. This assay measures the charge flow into and out of an electrode sealed on the surface of a cell. The flow of chloride ions entering the cell is measured as a function of the current that leaves the cell to maintain electrical equilibrium within the cell as the gate opens. The patch assay is fully described in Hammill, O.P. et al., Improved Patch Clamp Techniques for High Resolution Current Recording from Cells and Cell-Free Membrane Patches, Pfluegers Arch., 391:85-100 (1981).

Using the patch clamp assay, the effect a compound has on a receptor incorporated in the stable cell line can be determined. The range of sensitivity of the whole cell patch clamp assay is about 1000 pA and allows for the distinguishing of currents of between 2 and 5 pA.

The polynucleotides or polypeptides of the present invention can also be used in a method of screening drugs to identify those which enhance or block the interaction of GABA to the GABA_{A} receptor epsilon subunit. As an example, DNA encoding functional GABA_{A} receptor epsilon subunits are expressed in *Xenopus* oocytes. Expression is achieved by nuclear injection of recombinant CDM8 vectors in which the cloned cDNAs are under the transcriptional control of the cytomegalovirus promoter. The GABA_{A} receptor is then expressed on the outer surface of the oocyte and the oocyte is incubated under appropriate conditions for binding with GABA and a potential drug. The interaction of GABA with the GABA_{A} receptor epsilon subunit can then be measured in the presence of the drug and a determination could be made if the drug effected this interaction.

Potential antagonists include an antibody or in some cases, an oligonucleotide, which binds to the GABA_{A} receptor epsilon subunit to block its accessibility to GABA. An antagonist may also be a closely related protein of GABA which recognizes and binds to the GABA_{A} epsilon subunit but is an inactive form of GABA and effectively blocks the GABA_{A} receptor epsilon subunit.

Potential antagonists also include antisense constructs. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of GABA_{A} receptor epsilon subunit polypeptide. The antisense RNA oligonucleotide hybridizes to the mRNA *in vivo* and blocks translation of the mRNA molecule into the GABA_{A} receptor epsilon subunit polypeptide (antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of GABA_{A} receptor epsilon subunit polypeptide.

The agonists and antagonists identified above may be employed as therapeutic agents. Agonists are used to mimic the effects of GABA at the GABA_{A} receptor and therefore increase the inhibitory effects. These agonists are therefore useful for treating anxiety, Huntington's Chorea, muscular spasms and rigidity, and sleep and seizure disorders. The antagonists block the inhibition mediated by the receptor and therefore increase neuronal firing and are therefore useful for the treatment of Alzheimer's disease, Parkinson's disease and overdoses with benzodiazepine. Antagonists may also be employed for enhancing cognition and for reversing sedation after application of general anesthesia during surgery.

The agonists and antagonists of the present invention may be employed in combination with a suitable pharmaceutical carrier. Such compositions comprise a therapeutically effective amount of the agonist or antagonist, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

The pharmaceutical compositions may be administered in a convenient manner such as by the oral, topical, intravenous, intraperitoneal, intramuscular, subcutaneous, intranasal or intradermal routes, including intra-anal for specifically treating epilepsy. The pharmaceutical compositions are administered in an amount which is effective for treating and/or prophylaxis of the specific indication. The amounts and dosage regimens of the compositions administered to a subject will depend on a number of factors such as the mode of administration, the nature of the condition being treated and the judgment of the prescribing physician. In general, the compositions are administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc.

The agonists or antagonists which are polypeptides may also be used for the preparation of a pharmaceutical composition which allows for expression of such polypeptides *in vivo*, which is often referred to as "gene therapy."

Thus, for example, such a pharmaceutical composition may be designed in a manner that it allows for engineering cells from a patient with a polynucleotide (DNA or RNA) encoding a polypeptide *ex vivo,* with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, the pharmaceutical composition may be designed so as to allow for engineering cells *in vivo* for expression of a polypeptide *in vivo* by, for example, procedures known in the art. As known in the art, a producer cell for producing a retroviral particle containing RNA encoding the polypeptide of the present invention may be present in such a pharmaceutical composition for administration to a patient for engineering cells *in vivo* and expression of the polypeptide *in vivo*. These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease. Briefly, sequences can be mapped to chromosomes by preparing PCR primers (preferably 15-25 bp) from the cDNA. Computer analysis of the cDNA is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. FISH requires use of the clones from which the EST was derived, and the longer the better. For example, 2,000 bp is good, 4,000 is better, and more than 4,000 is probably not necessary to get good results a reasonable percentage of the time. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al.*, Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units to T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973).

### Example 1

### Expression of Recombinant GABA_{A} receptor epsilon subunit in HEK 293 cells

A fragment of the cDNA encoding GABA_{A} receptor epsilon subunit was cloned into a vector using the CMV immediate early promoter to drive transcription of the gene. The vector, pCDM8, is available from by Invitrogen Corporation, San Diego, Calif. 92121. The cDNA fragment was inserted into the plasmid by first digesting the plasmid with HindIII and XbaI (blunted) and ligating the cDNA at those sites in the plasmid. Plasmid DNA was prepared by the alkaline lysis procedure (Maniatis, T., E.F. Fritsch, and J. Sambrook, 1989, Molecular Cloning: A Laboratory Manual. Cold Spring Harbor Laboratory, Cold Spring Harbor, N.Y.) followed by two cycles of CsCl equilibrium density gradient centrifugation.

Transfection protocol used to transfect HEK 293 cells (HeLa cells may also be transfected) was adapted from Chen, C., and H. Okayama, 1987. High Efficiency Transformation of Mammalian Cells by Plasmid DNA. Mol. Cell. Biol. 7:2745-2752 (Chan-Okayama CaPO₄ pH 6.95 techniques). A stock solution of 2.5M CaCl₂ (Mallinckrodt) was prepared, filter sterilized through a 0.45 µm-pore-size nitrocellulose filter (Nalge) and stored at -20 degree(s) C. Then 2X Bes-buffered saline (2XBBS) containing 50 mM Bes (pH 6.95), 280 mM NaCl, and 1.5 mM Na₂ HP04 was prepared, filter sterilized, and stored at -20 degree(s) C. N,N-bis(2-hydroxyethyl)-2-aminoetrane-sulfonic acid (BES) was obtained from Sigma. The pH was adjusted with HCl at room temperature. HEK293 cells (American Type Culture Collection, CRL 1573) were maintained in MEM (Gibco) supplemented with 10% Fetal Bovine Serum (Gibco), penicillin-streptomycin (Gibco), and glutamine (Gibco), in a 5% CO₂, 37 degree(s) C. Forma Scientific Incubator.

Exponentially growing HEK 293 cells were removed with trypsin-EDTA, plated at a density of 1.5X10⁶ cells per 10 cm plate, and incubated overnight at 5% CO₂, 37 degree(s) C in 10 mls of growth medium. For selection purposes 1 µg of a plasmid pWL0Neo (Stratagene) containing the aminoglycoside phosphotransferase (neo) gene driven by the thymidine kinase promoter was used per 10 cm dish containing 1X10⁶ cells. Plasmid DNA was mixed with 50 mu l of 2.5M CaCl₂ and brought to a final volume of 0.5 ml with water. This mixture was added to 0.5 ml of 2X BBS and incubated for 90 seconds at room temperature. This resulting calcium phosphate-DNA solution was added (1 ml volume) to the plate of cells, swirled gently, and incubated for 15-24 hours at 37 degree(s) C. under 3% CO₂. The media was removed, the cells were rinsed two time with MEM media, and rinsed with fresh MEM+10% Fetal Bovine Serum. The cells were incubated for 48 hours at 37 degree(s) C under 5% CO₂.

Forty-eight hours after transfection the cells were trypsenized and split (1:8) into four plates. They were grown under selective pressure of growth media containing 1 mg/ml G418 sulfate (Gibco) at 1 mg/ml and grown for 2 weeks until colonies appeared. Selection of stable transformants took approximately 15-20 days. Colonies were separated into 24 well dishes and used after sufficient growth in G418 selective media to seed two 10 cm dishes. After sufficient growth in the 10 cm dishes, one was used to prepare total RNA; cells in the remaining dish were cryopreserved for possible future plating. The RNA was analyzed by Northern blotting.

To determine the frequency of stable transformation, the cells were transfected, as described. At the point when the cells were split and plated under selective pressure (G418 sulfate), cells were also plated, in duplicate, at a density of 1-3X10³ cells per 10 cm plate. One set of plates was maintained in nonselective growth medium, while the duplicate plates were grown in growth media with G418 sulfate (a mg/ml). Control plates were maintained in nonselective media 5-7 days; the colonies were then stained and counted. To determine the number of Neo⁺ transformants, cells were maintained under selective pressure for 2-3 weeks; the colonies were then stained and counted. The transformation efficiency is expressed as a % transformation. This is determined by dividing the number of Neo^{r} colonies by the number of colonies grown in nonselective medium and multiplying the result by 100.

Clones from the transfected plasmids were tested for the expression of functional channels. These tests were carried out by patch-clamp electrophysiology of whole cells. The whole-cell configuration of patch clamp technique was used to record the GABA-mediated Cl-currents in human embryonic kidney cells transfected with GABA_{A} receptor epsilon subunits. Patch pipettes were made of borosilicate glass with a resistance of 0.5 to 2 megaohms. The cell was bathed in the buffer medium containing (mM) NaCl 135, KCl 5, MgCl₂ 1, CaCl₂ 1.8 and Hepes 5, pH 7.2. The pipette was filled with the solution containing (mM) CsCl140, EDTA 11, ATP 2, MgCl₂ 4 and Hepes 10, pH 7.3. The holding potential was -60 mV. The bathing solution containing 5 µM GABA_{A} with or without test drug was applied to the cell through a U-tube positioned about 100 microns away from the cell.

### Example 2

### Expression pattern of GABA_{A} Epsilon in human tissue

Northern blot analysis was carried out to examine the levels of expression of GABA_{A} epsilon in human tissues. Total cellular RNA samples were isolated with RNAzol™ B system (Biotecx Laboratories, Inc. 6023 South Loop East, Houston, TX 77033). About 10µg of total RNA isolated from each human tissue specified was separated on 1% agarose gel and blotted onto a nylon filter. (Sambrook, Fritsch, and Maniatis, Molecular Cloning, Cold Spring Harbor Press, (1989)). The labeling reaction was done according to the Stratagene Prime-It kit with 50ng DNA fragment. The labeled DNA was purified with a Select-G-50 column. (5 Prime - 3 Prime, Inc. 5603 Arapahoe Road, Boulder, CO 80303). The filter was then hybridized with radioactive labeled full length GABA_{A} Epsilon gene at 1,000,000 cpm/ml in 0.5 M NaPO₄, pH 7.4 and 7% SDS overnight at 65°C. After wash twice at room temperature and twice at 60°C with 0.5 x SSC, 0.1% SDS, the filter was then exposed at -70°C overnight with an intensifying screen.

### Example 3

### Baculovirus expression of GABA_{A} Receptor Epsilon Subunit

A general method for expression of other GABA_{A} receptor subunits has been described previoulsy (Carter, D.B., et al., Bio/Technology, 10:679-681 (1992)). In this example, SF-9 cells (approximately 10⁶ cells) are infected with baculovirus constructs (approximately 10⁸ pfu), which express the epsilon subunit. Cells might also be co-infected with combinations of baculovirus constructs that express the epsilon subunit and other known GABA_{A} receptor subunits. The infected SF-9 cells are harvested after 60 hours. The cells are used for electrophysiological recordings (see example 1) or for radioligand binding assays. For the latter, cells are broken in a solution containing 118 mM NaCl, 5 mM KCl, 20 mM HEPES-Tris, pH 7,3, with a Polytron homogenizer. Unbroken cells and nuclei aggregates are removed by centrifugation at 1000g for 10 min. Cell membranes are then recovered by centrigugation of the supernatant at 40000g for 50 min. The pellet is resuspended in a solution containing 300 mM sucrose, 5 mM Tris-HCl, pH 7.5 and 20 % glycerol. The membranes are frozen at -80 degrees C until used for radioligand binding assays.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides encoding at least the mature form of the polypeptide having the deduced amino acid sequence as shown in SEQ ID NO:2;
(b) polynucleotides having the coding sequence as shown in SEQ ID NO:1 encoding at least the mature form of the polypeptide;
(c) polynucleotides encoding the polypeptide having the amino acid sequence of at least the mature form of the polypeptide encoded by the cDNA contained in ATCC 75810;
(d) polynucleotides having the coding sequence of the cDNA contained in ATCC 75810 encoding at least the mature form of the polypeptide;
(e) polynucleotides encoding a fragment of a polypeptide encoded by a polynucleotide of any one of (a) to (d) wherein said fragment has the activity of a GABA_{A} receptor epsilon subunit polypeptide as encoded by a polynucleotide of any one of (a) to (d) and/or binds an antibody that specifically binds to a polypeptide having the sequence as shown in SEQ ID NO:2; and
(f) polynucleotides the sequence of which has at least 95% sequence identity to a polynucleotide as defined in any one of (a) to (d);
or the complementary strand of such a polynucleotide.

2. The polynucleotide of claim 1 which is DNA.

3. The DNA of claim 2 which is genomic DNA.

4. The polynucleotide of claim 1 which is RNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell genetically engineered with the polynucleotide of any one of claims 1 to 4 or with the vector of claim 5 or 6.

8. A process for producing a polypeptide encoded by a polynucleotide of any one of claims 1 to 4 comprising: culturing the host cell of claim 7 and recovering the polypeptide encoded by said polynucleotide from the culture.

9. A process for producing cells capable of expressing a polypeptide encoded by a polynucleotide of any one of claims 1 to 4 comprising genetically engineering cells with the vector of claim 5 or 6.

10. A polypeptide having the amino acid sequence encoded by a polynucleotide of any one of claims 1 to 4 or obtainable by the process of claim 8 wherein said polypeptide is encoded by a polynucleotide of any one of claims 1 to 4 contained in said host cell.

11. An antibody specific for the polypeptide of claim 10.

12. The antibody of claim 11, which is selected from the group consisting of:
(a) a humanized antibody;
(b) a single chain antibody;
(c) a polyclonal antibody;
(d) a monoclonal antibody; and
(e) a Fab fragment.

13. A nucleic acid molecule which specifically hybridizes under stringent conditions to a polynucleotide of any one of claims 1 to 4.

14. An antagonist/inhibitor against the polypeptide of claim 10, which is an antibody according to claim 11 or 12 or an antisense construct which is specific for the polynucleotide of claim 1.

15. A pharmaceutical composition comprising the polynucleotide of any one of claims 1 to 4, the polypeptide of claim 10 or the antagonist/inhibitor of claim 14 and optionally a pharmaceutical acceptable carrier.

16. A diagnostic composition comprising the polynucleotide of any one of claims 1 to 4 or the nucleic acid molecule of claim 13.

17. Use of the polypeptide of claim 10 or of the polynucleotide of any one of claims 1 to 4 for the preparation of a pharmaceutical composition for the treatment of anxiety, Huntington's Chorea, muscular spasms and rigidity or sleep and seizure disorders.

18. Use of the antagonist/inhibitor of claim 14 for the preparation of a pharmaceutical composition for the treatment of Alzheimer's disease, Parkinson's disease, overdoses with benzodiazepine, for enhancing cognition or for reversing sedation after application of general anesthesia during surgery.

## Patentansprüche

1. Polynucleotid ausgewählt aus der Gruppe bestehend aus
(a) Polynucleotiden, die zumindest die reife Form des Polypeptids codieren, das die abgeleitete Aminosäuresequenz wie in SEQ ID NO:2 gezeigt aufweist;
(b) Polynucleotiden, die die codierende Sequenz wie in SEQ ID NO:1 gezeigt aufweisen, die zumindest die reife Form des Polypeptids codieren;
(c) Polynucleotiden, die das Polypeptid codieren, das die Aminosäuresequenz von zumindest der reifen Form des Polypeptids aufweist, das von der in ATCC 75810 enthaltenen cDNA codiert wird;
(d) Polynucleotiden, die die codierende Sequenz der in ATCC 75810 enthaltenen cDNA aufweisen, die zumindest die reife Form des Polypeptids codiert;
(e) Polynucleotiden, die ein Fragment eines Polypeptids, das von einem Polynucleotid gemäß (a) bis (d) codiert wird, codieren, wobei das Fragment die Aktivität der Epsilon-Untereinheit eines GABA_{A}-Rezeptor-Polypeptids, wie sie von einem Polynucleotid gemäß (a) bis (d) codiert wird, aufweist und/oder einen Antikörper bindet, der ein Polypeptid spezifisch bindet, das die Sequenz wie in SEQ ID NO:2 gezeigt aufweist; und
(f) Polynucleotiden, deren Sequenz zumindest 95% Sequenzidentität aufweist mit einem Polynucleotid wie definiert in (a) bis (d);
oder der komplementäre Strang eines solchen Polynucleotids.

2. Polynucleotid nach Anspruch 1, das DNA ist.

3. DNA nach Anspruch 2, die genomische DNA ist.

4. Polynucleotid nach Anspruch 1, das RNA ist.

5. Vektor enthaltend das Polynucleotid nach einem der Ansprüche 1 bis 4.

6. Vektor nach Anspruch 5, in dem das Polynucleotid funktionell verbunden ist mit Expressionskontrollsequenzen, die die Expression in prokaryontischen oder eukaryontischen Wirtszellen erlauben.

7. Wirtszelle, die genetisch modifiziert ist mit dem Polynucleotid nach einem der Ansprüche 1 bis 4 oder mit dem Vektor nach Anspruch 5 oder 6.

8. Verfahren zur Herstellung eines Polypeptids, das von einem Polynucleotid nach einem der Ansprüche 1 bis 4 codiert wird, umfassend: Züchten der Wirtszelle nach Anspruch 7 und Gewinnung des Polypeptids, das von dem Polynucleotid codiert wird, aus der Kultur.

9. Verfahren zur Herstellung von Zellen, die in der Lage sind, ein Polypeptid, das von einem Polynucleotid nach einem der Ansprüche 1 bis 4 codiert wird, zu exprimieren, umfassend das genetische Modifizieren von Zellen mit einem Vektor nach Anspruch 5 oder 6.

10. Polypeptid, das die Aminosäuresequenz aufweist, die von einem Polynucleotid nach einem der Ansprüche 1 bis 4 codiert wird, oder erhältlich ist durch das Verfahren nach Anspruch 8, wobei das Polypeptid codiert wird von einem Polynucleotid nach einem der Ansprüche von 1 bis 4, das in der Wirtszelle enthalten ist.

11. Antikörper spezifisch für das Polypeptid nach Anspruch 10.

12. Antikörper nach Anspruch 11, der ausgewählt ist aus der Gruppe bestehend aus:
(a) einem humanisierten Antikörper;
(b) einem Einzelkettenantikörper;
(c) einem polyclonalen Antikörper;
(d) einem monoclonalen Antikörper; und
(e) einem Fab-Fragment.

13. Nucleinsäuremolekül, das unter stringenten Bedingungen mit einem Polynucleotid nach einem der Ansprüche 1 bis 4 spezifisch hybridisiert.

14. Antagonist/Inhibitor gegen das Polypeptid nach Anspruch 10, der ein Antikörper nach Anspruch 11 oder 12 oder ein Antisense-Konstrukt ist, das spezifisch ist für das Polynucleotid nach Anspruch 1.

15. Arzneimittel umfassend das Polynucleotid nach einem der Ansprüche 1 bis 4, das Polypeptid nach Anspruch 10 oder den Antagonist/Inhibitor nach Anspruch 14 und gegebenenfalls einen pharmazeutisch verträglichen Träger.

16. Diagnostische Zusammensetzung umfassend das Polynucleotid nach einem der Ansprüche 1 bis 4 oder das Nucleinsäuremolekül nach Anspruch 13.

17. Verwendung des Polypeptids nach Anspruch 10 oder des Polynucleotids nach einem der Ansprüche 1 bis 4 für die Herstellung eines Arzneimittels zur Behandlung von Angstzuständen, Chorea Huntington, Muskelkrämpfen und - starre oder Schlafstörungen und Krampfanfall-auslösender Erkrankung.

18. Verwendung des Antagonisten/Inhibitors nach Anspruch 14 zur Herstellung eines Arzneimittels zur Behandlung von Alzheimer-Krankheit, Parkinson-Krankheit, Überdosen von Benzodiazepin, zur Steigerung des Wahrnehmungsvermögens, oder zur Aufhebung von Sedierung nach Anwendung allgemeiner Anästhesie bei einem chirurgischen Eingriff.

## Revendications

1. Polynucléotide sélectionné dans le groupe comprenant
(a) des polynucléotides codant au moins la forme mature du polypeptide ayant la séquence aminoacide déduite telle que présentée dans SEQ ID NO:2 ;
(b) des polynucléotides ayant la séquence codante telle que présentée dans SEQ ID NO:1 codant au moins la forme mature du polypeptide ;
(c) des polynucléotides codant le polypeptide ayant la séquence aminoacide d'au moins la forme mature du polypeptide codé par l'ADNc contenu dans ATCC 75810 ;
(d) des polynucléotides ayant la séquence codante de l'ADNc contenu dans ATCC75810 codant au moins la forme mature du polypeptide ;
(e) des polynucléotides codant un fragment d'un polypeptide codé par un polynucléotide de l'un quelconque des (a) à (d), dans lesquels ledit fragment a l'activité d'un polypeptide à sous-unité epsilon récepteur GABA_{A} tel que codé par un polynucléotide de l'un quelconque des (a) à (d) et/ou lie un anticorps qui se lie spécifiquement à un polypeptide ayant la séquence telle que présentée dans SEQ ID NO:2 ; et
(f) des polynucléotides dont la séquence a au moins 95% d'identité de séquence avec un polynucléotide tel que défini dans l'un quelconque des (a) à (d) ;
ou le brin complémentaire d'un tel polynucléotide.

2. Polynucléotide de la revendication 1 qui est de l'ADN.

3. ADN de la revendication 2 qui est de l'ADN génomique.

4. Polynucléotide de la revendication 1 qui est l'ARN.

5. Vecteur contenant le polynucléotide de l'une quelconque des revendications 1 à 4.

6. Vecteur de la revendication 5, dans lequel le polynucléotide est lié de manière opérationnelle à des séquences de contrôle d'expression permettant l'expression dans des cellules hôtes procaryotes ou eucaryotes.

7. Cellule hôte génétiquement modifiée avec le polynucléotide de l'une quelconque des revendications 1 à 4 ou avec le vecteur de la revendication 5 ou 6.

8. Procédé de production d'un polypeptide codé par un polynucléotide de l'une quelconque des revendications 1 à 4 comprenant : la culture de la cellule hôte de la revendication 7 et la récupération du polypeptide codé par ledit polynucléotide de la culture.

9. Procédé de production de cellules capables d'exprimer un polypeptide codé par un polynucléotide de l'une quelconque des revendications 1 à 4 comprenant la modification génétique de cellules avec le vecteur de la revendication 5 ou 6.

10. Polypeptide ayant la séquence aminoacide codée par un polynucléotide de l'une quelconque des revendications 1 à 4 ou susceptible d'être obtenu par le procédé de la revendication 8, dans lequel ledit polypeptide est codé par un polynucléotide de l'une quelconque des revendications 1 à 4 contenu dans ladite cellule hôte.

11. Anticorps spécifique au polypeptide de la revendication 10.

12. Anticorps de la revendication 11 qui est sélectionné dans le groupe comprenant :
(a) un anticorps humanisé ;
(b) un anticorps à chaîne simple ;
(c) un anticorps polyclonal ;
(d) un anticorps monoclonal ; et
(e) un fragment Fab.

13. Molécule d'acide nucléique qui s'hybride spécifiquement sous des conditions stringentes à un polynucléotide de l'une quelconque des revendications 1 à 4.

14. Antagoniste/inhibiteur contre le polypeptide de la revendication 10, qui est un anticorps selon la revendication 11 ou 12 ou une construction anti-sens qui est spécifique au polynucléotide de la revendication 1.

15. Composition pharmaceutique comprenant le polynucléotide de l'une quelconque des revendications 1 à 4, le polypeptide de la revendication 10 ou l'antagoniste/inhibiteur de la revendication 14 et facultativement un porteur acceptable en pharmaceutique.

16. Composition diagnostique comprenant le polynucléotide de l'une quelconque des revendications 1 à 4 ou la molécule d'acide nucléique de la revendication 13.

17. Utilisation du polypeptide de la revendication 10 ou du polynucléotide de l'une quelconque des revendications 1 à 4 pour la préparation d'une composition pharmaceutique pour le traitement de l'anxiété, de la Chorée de Huntington, des spasmes ou de la rigidité musculaires ou des troubles du sommeil et de crise.

18. Utilisation de l'antagoniste/inhibiteur de la revendication 14 pour la préparation d'une composition pharmaceutique pour le traitement de la maladie d'Alzheimer, de la maladie de Parkinson, des overdoses à la benzodiazépine, pour augmenter la cognition ou pour inverser la sédation après application d'anesthésie générale pendant la chirurgie.


### SEQUENCE LISTING

(1) GENERAL INFORMATION:
   (i) APPLICANT: KIRKNESS, ET AL.
   (ii) TITLE OF INVENTION: GABA_{A} Receptor Epsilon Subunit
   (iii) NUMBER OF SEQUENCES: 2
   (iv) CORRESPONDENCE ADDRESS:
      (A) ADDRESSEE: CARELLA, BYRNE, BAIN, GILFILLAN, CECCHI, STEWART & OLSTEIN
      (B) STREET: 6 BECKER FARM ROAD
      (C) CITY: ROSELAND
      (D) STATE: NEW JERSEY
      (E) COUNTRY: USA
      (F) ZIP: 07068
   (v) COMPUTER READABLE FORM:
      (A) MEDIUM TYPE: 3.5 INCH DISKETTE
      (B) COMPUTER: IBM PS/2
      (C) OPERATING SYSTEM: MS-DOS
      (D) SOFTWARE: WORD PERFECT 5.1
   (vi) CURRENT APPLICATION DATA:
      (A) APPLICATION NUMBER:
      (B) FILING DATE: Submitted herewith
      (C) CLASSIFICATION:
   (vii) PRIOR APPLICATION DATA
      (A) APPLICATION NUMBER:
      (B) FILING DATE:
   (viii) ATTORNEY/AGENT INFORMATION:
      (A) NAME: FERRARO, GREGORY D.
      (B) REGISTRATION NUMBER: 36,134
      (C) REFERENCE/DOCKET NUMBER: 325800-188
   (ix) TELECOMMUNICATION INFORMATION:
      (A) TELEPHONE: 201-994-1700
      (B) TELEFAX: 201-994-1744
(2) INFORMATION FOR SEQ ID NO:1:
   (i) SEQUENCE CHARACTERISTICS
      (A) LENGTH: 1650 BASE PAIRS
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:1:
   GGGACAGGGC TGAGGATGAG GAGAACCCTG GGGACCCAGA AGACCGTGCC TTGCCCGGAA   60
   GTCCTGCCTG TAGGCCTGAA GGACTTGCCC TAACAGAGCC TCAACAACTA CCTGGTGATT  120
   CCTACTTCAG CCCCTTGGTG TGAGCAGCTT CTCAACATGA ACTACAGCCT CCACTTGGCC  180
   TTCGTGTGTC TGAGTCTCTT CACTGAGAGG ATGTGCATCC AGGGGAGTCA GTTCAACGTC  240
   GAGGTCGGCA GAAGTGACAA GCTTTCCCTG CCTGGCTTTG AGAACCTCAC AGCAGGATAT  300
   AACAAATTTC TCAGGCCCAA TTTTGGTGGA GAACCCGTAC AGATAGCGCT GACTCTGGAC  360
   ATTGCAAGTA TCTCTAGCAT TTCAGAGAGT AACATGGACT ACACAGCCAC CATATACCTC  420
   CGACAGCGCT GGATGGACCA GCGGCTGGTG TTTGAAGGCA ACAAGAGCTT CACTCTGGAT  480
   GCCCGCCTCG TGGAGTTCCT CTGGGTGCCA GATACTTACA TTGTGGAGTC CAAGAAGTCC  540
   TTCCTCCATG AAGTCACTGT GGGAAACAGG CTCATCCGCC TCTTCTCCAA TGGCACGGTC  600
   CTGTATGCCC TCAGAATCAC GACAACTGTT GCATGTAACA TGGATCTGTC TAAATACCCC  660
   ATGGACACAC AGACATGCAA GTTGCAGCTG GAAAGCTGGG GCTATGATGG AAATGATGTG  720
   GAGTTCACCT GGCTGAGAGG GAACGACTCT GTGCGTGGAC TGGAACACCT GCGGCTTGCT  780
   CAGTACACCA TAGAGCGGTA TTTCACCTTA GTCACCAGAT CGCAGCAGGA GACAGGAAAT  840
   TACACTAGAT TGGTCTTACA GTTTGAGCTT CGGAGGAATG TTCTGTATTT CATTTTGGAA  900
   ACCTACGTTC CTTCCACTTT CCTGGTGGTG TTGTCCTGGG TTTCATTTTG GATCTCTCTC  960
   GATTCAGTCC CTGCAAGAAC CTGCATTGGA GTGACGACCG TGTTATCAAT GACCACACTG 1020
   ATGATCGGGT CCCGCACTTC TCTTCCCAAC ACCAACTGCT TCATCAAGGC CATCGATGTG 1080
   TACCTGGGGA TCTGCTTTAG CTTTGTGTTT GGGGCCTTGC TAGAATATGC AGTTGCTCAC 1140
   TACAGTTCCT TACAGCAGAT GGCAGCCAAA GATAGGGGGA CAACAAAGGA AGTAGAAGAA 1200
   GTCAGTATTA CTAATATCAT CAACAGCTCC ATCTCCAGCT TTAAACGGAA GATCAGCTTT 1260
   GCCAGCATTG AAATTTCCAG CGACAACGTT GACTACAGTG ACTTGACAAT GAAAACCAGC 1320
   GACAAGTTCA AGTTTGTCTT CCGAGAAAAG ATGGGCAGGA TTGTTGATTA TTTCACAATT 1380
   CAAAACCCCA GTAATGTTGA TCACTATTCC AAACTACTGT TTCCTTTGAT TTTTATGCTA 1440
   GCCAATGTAT TTTACTGGGC ATACTACATG TATTTTTGAG TCAATGTTAA ATTTCTTGCA 1500
   TGCCATAGGT CTTCAACAGG ACAAGATAAT GATGTAAATG GTATTTTAGG CCAAGTGTGC 1560
   ACCCACATCC AATGGTGCTA CAAGTGACTG AAATAATATT TGAGTCTTTC TGCTCAAAGA 1620
   ATGAAGCTCC AACCATTGTT CTAAGCTGTG                                  1650
(2) INFORMATION FOR SEQ ID NO:2:
   (i) SEQUENCE CHARACTERISTICS
      (A) LENGTH: 440 AMINO ACIDS
      (B) TYPE: AMINO ACID
      (C) STRANDEDNESS:
      (D) TOPOLOGY: LINEAR
   (ii) MOLECULE TYPE: PROTEIN
   (xi) SEQUENCE DESCRIPTION: SEQ ID NO:2:
   Met Asn Tyr Ser Leu His Leu Ala Phe Val Cys Leu Ser Leu Phe
                   -20                     -15                -10
   Thr Glu Arg Met Cys Ile Gln Gly Ser Gln Phe Asn Val Glu Val
                   -5                          1               5
   Gly Arg Ser Asp Lys Leu Ser Leu Pro Gly Phe Glu Asn Leu Thr
               10                  15                  20
   Ala Gly Tyr Asn Lys Phe Leu Arg Pro Asn Phe Gly Gly Glu Pro
               25                  30                  35
   Val Gln Ile Ala Leu Thr Leu Asp Ile Ala Ser Ile Ser Ser Ile
               40                  45                  50
   Ser Glu Ser Asn Met Asp Tyr Thr Ala Thr Ile Tyr Leu Arg Gln
               55                  60                  65
   Arg Trp Met Asp Gln Arg Leu Val Phe Glu Gly Asn Lys Ser Phe
               70                  75                  80
   Thr Leu Asp Ala Arg Leu Val Glu Phe Leu Trp Val Pro Asp Thr
   85 90 95
   Tyr Ile Val Glu Ser Lys Lys Ser Phe Leu His Glu Val Thr Val
   100 105 110
   Gly Asn Arg Leu Ile Arg Leu Phe Ser Asn Gly Thr Val Leu Tyr
   115 120 125
   Ala Leu Arg Ile Thr Thr Thr Val Ala Cys Asn Met Asp Leu Ser
   130 135 140
   Lys Tyr Pro Met Asp Thr Gln Thr Cys Lys Leu Gln Leu Glu Ser
   145 150 155
   Trp Gly Tyr Asp Gly Asn Asp Val Glu Phe Thr Trp Leu Arg Gly
   160 165 170
   Asn Asp Ser Val Arg Gly Leu Glu His Leu Arg Leu Ala Gln Tyr
   175 180 185
   Thr Ile Glu Arg Tyr Phe Thr Leu Val Thr Arg Ser Gln Gln Glu
   190 195 200
   Thr Gly Asn Tyr Thr Arg Leu Val Leu Gln Phe Glu Leu Arg Arg
   205 210 215
   Asn Val Leu Tyr Phe Ile Leu Glu Thr Tyr Val Pro Ser Thr Phe
   220 225 230
   Leu Val Val Leu Ser Trp Val Ser Phe Trp Ile Ser Leu Asp Ser
   235 240 245
   Val Pro Ala Arg Thr Cys Ile Gly Val Thr Thr Val Leu Ser Met
   250 255 260
   Thr Thr Leu Met Ile Gly Ser Arg Thr Ser Leu Pro Asn Thr Asn
   265 270 275
   Cys Phe Ile Lys Ala Ile Asp Val Tyr Leu Gly Ile Cys Phe Ser
   280 285 290
   Phe Val Phe Gly Ala Leu Leu Glu Tyr Ala Val Ala His Tyr Ser
   295 300 305
   Ser Leu Gln Gln Met Ala Ala Lys Asp Arg Gly Thr Thr Lys Glu
   310 315 320
   Val Glu Glu Val Ser Ile Thr Asn Ile Ile Asn Ser Ser Ile Ser
   325 330 335
   Ser Phe Lys Arg Lys Ile Ser Phe Ala Ser Ile Glu Ile Ser Ser
   340 345 350
   Asp Asn Val Asp Tyr Ser Asp Leu Thr Met Lys Thr Ser Asp Lys
   355 360 365
   Phe Lys Phe Val Phe Arg Glu Lys Met Gly Arg Ile Val Asp Tyr
   370 375 380
   Phe Thr Ile Gln Asn Pro Ser Asn Val Asp His Tyr Ser Lys Leu
   385 390 395
   Leu Phe Pro Leu Ile Phe Met Leu Ala Asn Val Phe Tyr Trp Ala
   400 405 410
   Tyr Tyr Met Tyr Phe
   415